Europäisches Patentamt

European Patent Office

Office européen des brevets

(19)

(11) Publication number: **0 045 031**
**A1**

(12) **EUROPEAN PATENT APPLICATION**

(21) Application number: **81105696.9**

(22) Date of filing: **20.07.81**

(51) Int. Cl.³: **C 12 Q 1/00,** C 12 Q 1/44,
C 12 Q 1/48

(30) Priority: **22.07.80 US 171066**

(43) Date of publication of application: **03.02.82**
**Bulletin 82/5**

(84) Designated Contracting States: **BE DE FR GB IT LU NL SE**

(71) Applicant: **Baker Instruments Corporation, 2196 Avenue C. P.O. Box 2168, Bethlehem Pennsylvania 18001 (US)**

(72) Inventor: **Matteo, Martha R., Greenmeadow Road, Pleasantville N.Y. 10570 (US)**
Inventor: **Battey, York C., 119-29 180 Street, St. Albans N.Y. 11434 (US)**

(74) Representative: **Vossius.Vossius.Tauchner.Heunemann.Rauh, Siebertstrasse 4 P.O. Box 86 07 67, D-8000 München 86 (DE)**

(54) **Glycerol detection reagent and its use.**

(57) It is known to determine triglycerides concentration in serum by converting the triglycerides to glycerol and measuring the glycerol content. An improved glycerol detection reagent is disclosed and its use for measuring the triglyceride concentration of a substance.

EP 0 045 031 A1

Our Ref.: R 228 EP          July 20, 1981

Case:      BIC-1150

BAKER INSTRUMENTS CORPORATION

Bethlehem, PA, USA

" Glycerol Detection Reagent and its Use "

The invention relates to an improved glycerol detection reagent.

In many fields, it is important to determine triglyceride concentration of a substance. This is particularly important in clinical biochemistry. The determination of triglyceride concentration in human serum is an important part of a routine lipid analysis in clinical chemistry.

There are numerous prior art methods and compositions for measuring triglycerides concentration. One class of methods includes transforming the triglycerides into glycerol and then measuring the glycerol content.

Generally, a glycerol detection reagent is known in which the glycerol is phosphorylated with adenosine triphosphate in the presence of glycerol kinase to give glycerol-1-phosphate and adenosine diphosphate.

The adenosine diphosphate is then converted by phosphoenol pyruvate in the presence of pyruvate kinase into pyruvate and adenosine triphosphate. The pyruvate formed is hydrogenated by nicotinamide adenine dinucleotide in the reduced form in the presence of lactate dehydrogenase to obtain lactate while the nicotinamide adenine dinucleotide is oxidized to nicotinamide adenine dinucleotide. The amount of nicotinamide adenine dinucleotide utilized by the reaction is equivalent to the amount of glycerol and the nicotinamide adenine dinucleotide can easily be determined quantitatively on the basis of its absorption at 366, 340, or 344 nm.

Generally, a glycerol detection reagent can result in errors due to endogenous enzymes, endogenous substrates, and turbidity.

The instant invention overcomes these errors and also provides an extremely fast measurement. This last advantage is particularly important in connection with a fast acting formulation which will hydrolize the triglycerides to glycerol rapidly. Such a fast acting formulation is disclosed in the concurrently filed patent application having the same applicants and assignee as the instant application (corresponding to USSN 171,065).

In its broader scope, the invention features a glycerol detection reagent consisting essentially of glycerol kinase, pyruvate kinase, lactate dehydrogenase, adenosine triphosphate, phosphoenol pyruvate, nicotinamide adenine dinucleotide (reduced form), bovine serum albumen, magnesium sulphate, and a buffer; the ratio of the pyruvate kinase to lactate dehydrogenase is about 1:1; the ratio of pyruvate kinase and lactate dehydrogenase each to glycerol kinase is about 2:1; the magnesium ions are present in the range from about 3.1 to about 6.0 mM; and the pH of the reagent is in the range of from about 6.9 to about 7.6.

Preferably, the glycerol kinase, pyruvate kinase, and lactate dehydrogenase are lyophilized and the bovine serum albumen is crystallized and lyophilized.

Preferably, the buffer is in the form of phosphates such as potassium phosphate, monobasic and potassium phosphate, dibasic.

Illustrative non-limiting examples of the practice of the invention are set out below. Numerous other examples can readily be evolved in the light of the guiding principles and teachings contained herein. The examples given herein are intended merely to illustrate the invention and not in any sense to limit the manner in which the invention can be practiced.

Example 1

Example 1 was carried out to show that a glycerol detection system according to the invention could obtain an accurate determination rapidly.

The aforementioned concurrently filed patent (corresponding to USSN 171,065) application/discloses the use of at least one bacterial lipase derived from a bacterial source in the group

0045031

consisting of Pseudomonas species and Chromobacterium species. These lipases above or in combination with each other have been found to hydrolize triglycerides rapidly and completely to an endpoint in less than 4 minutes. The preferable lipase are from Pseudomonas fluorescens and Chromobacterium viscosum as well as a combination of these lipases. The Example 1 used a lipase ratio of Pseudomonas fluorescens to Chromobacterium viscosum of about 90:10 in the amount of 100 lipase units per mL of the composition.

As used herein, one lipase unit is defined as the amount which will release one micromole of fatty acid per minute from emulsified olive oil at 22°C and pH 7.5 in a reaction mixture as follows:

> 0.4 M sodium chloride
>
> 6 millimolar magnesium sulphate·7H$_2$0
>
> 5 millimolar calcium chloride·2H$_2$0
>
> 0.67 mg bovine serum albumen per milliliter
>     of the reaction mixture
>
> 137 mg olive oil in suspension per milliliter
>     of the reaction mixture

The glycerol detection reagent for the best mode was formulated as follows:

> a. All of the components for the reagent
>    were preweighed prior to formulation and
>    stored dessicated at appropriate temperatures.

b.  A potassium phosphate buffer was prepared
and the pH of the buffer was adjusted to be
about 7.5 at room temperature.

c.  Bovine serum albumen (crystallized,
lyophilized) was added to the buffer.

d.  The other components were then added to
the buffer as dry powders or as concentrated
solutions in the following order:

(i)  Adenosine triphosphate (ATP)

(ii) Phosphoenol pyruvate (PEP)

(iii) Nicotine Adenine Dinucleotide
(reduced form) (NADH)

(iv) $MgSO_4 \cdot 7H_2O$

(v) Glycerol Kinase (Lyophilized powder
preferred)

(vi) Pyruvate Kinase (lyophilized powder
preferred)

(vii) Lactate Dehydrogenase (lyophilized
powder preferred)

(viii) Selected lipase

The completed formulation was used immediately
after it was prepared or stored at 4°C for several
hours prior to its use.

The amounts of various components used in
the reagent are shown in Table 1:

0045031

## Table 1

| Component | Quantity per mL of Triglyceride Analysis Composition |
|---|---|
| ATP | 0.404 mg |
| PEP (monocyclohexyl ammonium salt) | 0.214 mg |
| NADH | 0.223 mg |
| $MgSO_4 \cdot 7H_2O$ | 0.764 mg |
| glycerol kinase | 2.1 IU |
| pyruvate kinase | 4.2 IU |
| lactate dehydrogenase | 4.1 IU |
| potassium phosphate (monobasic) | 1.16 mg |
| potassium phosphate (dibasic) | 10.103 mg |
| Bovine serum albumen | 3.40 mg |

An automated analyzer manufactured and marked by Union Carbide Corp. under the trademark CentrifiChem 400 System (Analyzer and Pipettor) was used. The instrument settings were such that the following parameters were obtained:

wavelength = 340 nm

temperature = $30^{\circ}C$

initial reading ($t_0$) = 15 seconds

final reading ($t_f$) = 4 minutes 15 seconds time interval = 4 minutes

serum volume = 5 $\mu$l

water added = 50 $\mu$l

triglyceride analyzer composition - 350 $\mu$l

The triglyceride concentration of each serum sample was calculated according to the formula:

$$\text{Triglycerides (mg/dL)} = (A_o - A_f) \div \varepsilon^{340}_{NADH} \times \frac{\text{av. mol.}}{\text{wt. trig.}} \times \frac{\text{dilution}}{\text{factor}} \times 100$$

$A_o$ = initial absorbance ($t_o$ = 15 seconds)

$A_f$ = final absorbance (4 min. 15 sec.)

$\varepsilon^{340}_{NADH}$ = 6.23 x $10^3$ abs. units $cm^2 mmol^{-1}$

dilution factor = 80 + 1 = 81

Performance characteristics using the Centrifichem 400 System are shown in Table 2 were achieved:

### Table 2

Precision

|  | No. of Replicates | Average Value $\bar{x}$(mg/dL) | Std. Deviation (mg/dL) | Coefficient of Variance (%) |
|---|---|---|---|---|
| **Within-Run Comparison** | | | | |
| Normal Control | 22 | 46.4 | 1.87 | 4.0 |
| Elevated Lipid Control | 23 | 141 | 3.0 | 2.2 |
| **Between-Run Comparison** | | | | |
| Normal Control | 115 | 49 | – | 3.0 |
| Elevated Lipid Control | 115 | 146 | – | 3.2 |

### Example 2

Tests were carried out to optimize and determine the range of the respective components of the instant glycerol detection reagent.

The following parameters were determined:

The ratio of the enzymes glycerol kinase, pyruvate kinase, and lactic dehydrogenase was found to be critical. The ratio of pyruvate kinase to lactic dehydrogenase should be about 1:1 and the ratio of glycerol kinase to either of the other enzymes should be about 1:2. Changes in these ratios affect linearity and sensitivity. A reduction in overall concentration will eventually reduce overall reaction rate.

For NADH, the initial absorbance reading at 340 nm determines the linearity. Freshly prepared composition has a theoretical absorbance value = 340 nm of 1.8, due to NADH. An absorbance value at 340 nm of 1.0 provides good linearity for triglycerides concentrations up to at least 1000 mg per dL serum.

Phosphate was selected as the buffer to minimize interference from serum-containing phosphatases. High levels of the phosphate buffer can tend to inhibit the glycerol detection reagent or produce a magnesium phosphate precipitation. Low levels can be insufficient to provide buffering. Other buffers can be used satisfactorily in accordance with prior art practice such as triethanolamine and HEPES (N-2-hydroxyethylpiperazine-N'-2-ethanesulfonic acid).

Magnesium sulphate in the range of from about 3.1 mM to about 6.0mM has been tested. The higher levels tended to result in a precipitation in the pre-

0045031

sence of $NH_4^+$.

A pH range of from about 6.9 to about 7.6 has been found acceptable within the constraint on the buffer given above. NADH stability tends to decrease for a pH below about 7. Preferably, the glycerol detection reagent should have a pH in the range of from about 7.5 to about 7.6.

Generally, any glycerol detection reagent for use with a spectrophotometer should take into account turbidity which does not change during the course of the reaction; endogenous substrates in sera such as glycerol and pyruvate; and endogenous enzymes in sera such as "phosphatases" which break down reagent components such as adensine triphosphate to adenosine diphosphate and phosphoenol pyruvate to pyruvate.

The instant glycerol detection reagent showed the following advantageous properties:

Turbidity-Static

In accordance with conventional practice, absorbance calculations are made from the difference in absorbance so that static turbidity effects are cancelled out.

Endogenous Substrates

Endogenous substrates react sunstantially with the instant glycerol detection reagent within the first 15 seconds before the initial absorbance measurement.

Endogenous Enzymes

Endogenous enzymes such as alkaline phosphatase
(up to about 1000 IU. of human serum alkaline
phosphatase) are not reactive with the instant
glycerol detection reagent and thus, do not
interface with the measurements of triglycerides
in sera.

Example 3

Example 3 was carried out to show that the
instant glycerol detection reagent is fast. For this
purpose, solutions of glycerol were prepared to correspond to triglycerides concentrations as follows:
50, 100, 200, 400, 600, 800, and 1000 mg triglycerides
per dL of serum. These solutions were based on the
assumption that 1 mg of glycerol is equivalent to
9.578 mg triglycerides.

The glycerol detection reagent of the Example 1
was reacted with each solution and the CentrifiChem 400
System was used.

Each reaction was at least about 90% completed
within 15 seconds.

We wish it to be understood that we do not
desire to be limited to the exact details described,
for obvious modifications will occur to a person
skilled in the art.

WHAT IS CLAIMED IS:

1.    A glycerol detection reagent consisting essentially of glycerol kinase, pyruvate kinase, lactate dehydrogenase, adenosine triphosphate, phosphoenol pyruvate, nicotinamide adenine dinucleotide (reduced form), bovine serum albumen, magnesium sulphate, and a buffer; the ratio of the pyruvate kinase to lactate dehydrogenase is about 1:1, the radio of pyruvate kinase and lactate dehydrogenase each to glycerol kinase is about 2:1; the magnesium ions are present in the range from about 3.1 to about 6.0 mM; and the pH of the reagent is in the range of from about 6.9 to about 7.6.

2.    The glycerol detection reagent of claim 1, wherein said glycerol kinase, pyruvate kinase, and lactate dehydrogenase are lyophilized, and said bovine serum albumen is crystallized and lyophilized.

3.    The glycerol detection reagent of claim 2, wherein said buffer is in the form of phosphates.

4.    The gylcerol detection reagent of claim 2, wherin said buffer comprises triethanolamine.

5.    The glycerol detection reagent of claim 2, wherein said buffer comprises N-2-hydroxyethylpiperazine-N'-2-ethanesulfonic acid.

6.    Use of the glycerol detection reagent according to any of claims 1 to 5 for measuring the triglyceride concentration of a substance.

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| X | US - A - 3 862 009 (WAHLEFELD et al.)<br><br>* Abstract; column 1, lines 1-6; column 2, lines 42-68; column 3, lines 8-38; claims 1,3,4,7, 9 * | 1,4,6 | C 12 Q 1/00<br>1/44<br>1/48 |
| | US - A - 4 066 508 (RAUSCHER et al.)<br><br>* Abstract; claims 10-12,14-16 * | 1,3,6 | |
| | US - A - 3 703 591 (BUCOLO GIO-VANNI)<br><br>* Abstract; column 2, lines 5-56; column 3, lines 5-46; claim 28 * | 1,3,6 | TECHNICAL FIELDS SEARCHED (Int. Cl.³)<br><br>C 12 Q 1/00<br>1/44<br>1/48 |
| | NATURE, vol. 196, 8th December 1962, pages 987-988 P.B. GARLAND et al.: "A rapid enzymatic assay for glycerol"<br><br>* Whole article * | 1,3 | |
| XE | DE - A - 3 006 789 (MILLIPORE CORP.)<br><br>* Claims 6-10; page 6, lines 1-19; examples 1,2 * | 1-3,6 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 06-11-1981 | DE LUCA |

EPO Form 1503.1  06.78